(19) Europäisches Patentamt
European Patent Office
Office européen des brevets

(11) Publication number: **0 102 935**
**B1**

(12) **EUROPEAN PATENT SPECIFICATION**

(45) Date of publication of patent specification: **26.02.86**

(51) Int. Cl.$^4$: **C 07 C 121/43**, C 07 C 120/00

(21) Application number: **83870085.4**

(22) Date of filing: **25.08.83**

(54) **Process for producing nitrilotriacetonitrile.**

(30) Priority: **30.08.82 US 412646**

(43) Date of publication of application:
**14.03.84 Bulletin 84/11**

(45) Publication of the grant of the patent:
**26.02.86 Bulletin 86/09**

(84) Designated Contracting States:
**BE DE FR GB IT NL**

(56) References cited:
**EP-A-0 102 343**
**GB-A-1 334 991**
**US-A-3 061 628**
**US-A-3 907 858**

(73) Proprietor: **MONSANTO COMPANY**
**Patent Department 800 North Lindbergh Boulevard**
**St. Louis, Missouri 63166 (US)**

(72) Inventor: **Shen, Chung Yu**
**12630 Conway Downs Drive**
**St. Louis Missouri 63141 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 102 935 B1**

**Description**

Background of the invention

Field of the invention

This invention relates to the production of nitrilotriacetonitrile, and more particularly to the source and manner of addition of hydrogen cyanide to the reaction mixture to produce nitrilo-triacetonitrile.

Background

It is well known that ammonia, an ammonium salt, or an ammonia-formaldehyde condensation product will react with formaldehyde and hydrogen cyanide to produce nitrilotriacetonitrile. A common ammonia-formaldehyde condensation product is hexamethylenetetramine (HMTA). These reactions have been generally defined in the following U.S. Patents to J. J. Singer, et al: 2,855,428, and 3,061,628 and to Morgan, et al: 3,463,805.

Nitrilotriacetonitrile can be produced from hexamethylenetetramine by the following reaction:

$$N_4(CH_2)_6+6CH_2O+12HCN \xrightarrow{pH\leq 1} 4N(CH_2CH)_3+6H_2O \quad (1)$$

If the hexamethylenetetramine is replaced by equivalent amounts of ammonia or ammonium and formaldehyde, nitrilotriacetonitrile can be produced from ammonia or an ammonium salt by the following reaction:

$$NH_3(\text{or } HN_4^+)+3CH_2O+3HCN \xrightarrow{pH\leq 1} \quad (2)$$

$$N(CH_2CN)_3(+H^+ \text{ if } NH_4^+ \text{ is used}$$

The effect of many factors on these reactions has been studied, including dilution ratios, temperature range; order of addition of reactants, order of reaction steps, and others. But, current processes use hydrogen cyanide as commercially available, which is anhydrous or substantially anhydrous form.

Hydrogen cyanide is most commonly produced commercially by reaction of ammonia with methane, methanol or other suitable volatile hydrocarbon, at elevated temperatures to produce hydrogen cyanide, hydrogen, and other inert gases.

Hydrogen cyanide is normally purified to greater than 98% purity and liquified prior to use.

This purification process typically comprises cooling the product gas stream; removing ammonia with a sulfuric acid solution or a mono-ammonium phosphate solution; absorbing hydrogen cyanide from the resulting gas stream in an acidic aqueous solution; stripping the hydrogen cyanide from the resulting solution; condensing the stripped hydrogen cyanide and water mixture; fractionating to produce hydrogen cyanide; liquifying the hydrogen cyanide, and stabilizing the liquified hydrogen cyanide to prevent polymerization.

Purification of hydrogen cyanide to the commercial grade anhydrous hydrogen cyanide as outlined above, is a complex process requiring large expenditures for equipment, energy, and material handling. Additionally, cyanide-containing waste streams are produced that must be treated and disposed of. It would be an advancement in the art to be able to use hydrogen cyanide to produce nitrilo-triacetonitrile without the costly and waste producing purification steps.

It is an advantage of this invention that nitrilotriacetonitrile can be produced using hydrogen cyanide without undergoing the usual purification.

It is a further advantage of this invention that the amount of waste in the hydrogen cyanide production process is reduced.

Further advantages can be seen from the following description and examples.

Summary of the invention

This invention provides a process for producing nitrilotriacetonitrile consisting essentially of:

a. producing a gas stream containing hydrogen cyanide;

b. cooling said gas to a temperature suitable for scrubbing;

c. scrubbing said cooled gas stream with a dilute aqueous solution of an inorganic acid to produce a hydrogen cyanide-containing solution;

d. adding formaldehyde and an ammonia derivative selected from the group consisting of ammonia, an ammonium derivative, and hexamethylenetetramine to said hydrogen cyanide-containing solution, to produce a reaction mixture; and

e. reacting said reaction mixture to produce nitrilotriacetonitrile.

Description of the preferred embodiment

Hydrogen cyanide can be produced by the reaction of methane, methanol, or other volatile hydrocarbon with ammonia in the presence of a catalyst or in a fluidized bed at a temperature in excess of 1000°C. The gas stream produced contains nitrogen, methane, ammonia, hydrogen, hydrogen cyanide, and other gas components. Unless otherwise specified, all parts and percents are by weight.

Hydrogen cyanide is produced commercially primarily by the Andrussow Process, the Degussa Process, also called the BMA Process, and the Fluohmic Process, and ammoxidation of methanol or other hydrocarbons. All of these processes use ammonia as the source of nitrogen, and the product gas stream will contain some unreacted ammonia. Since ammonia will react with hydrogen cyanide removal of this unreacted ammonia is an important step in the purification process.

The Andrussow Process produces hydrogen cyanide by the following theoretical reaction:

$$CH_4+NH_3+1.5O_2 \longrightarrow HCN+3H_2O \quad (3)$$

at about 1100°C, with heat being supplied by combustion of methane. Metal catalysts from Group VIII of the Periodic Table are normally used, preferably a Platinum-Rhodium gauze. The oxygen is normally supplied by mixing an appropriate amount of air with the other reactants. A typical product gas stream produced by the Andrussow Process has the following composition: nitrogen 64%, water 13%, hydrogen cyanide 11%, carbon monoxide 7%, hydrogen 2%, ammonia 2%, carbon dioxide 1%, methane 0.4%.

The Degussa Process produces hydrogen cyanide by the following theoretical reaction:

$$CH_4NH_3 \longrightarrow HCN+3H_2 \quad (4)$$

in a tubular reactor impregnated with a platinum catalyst at 1200°C, with heat being supplied by indirectly heating the tubes with gas or electricity. A typical product gas stream contains: hydrogen cyanide 71%, hydrogen 17% nitrogen 7%, methane 4%, ammonia 2%.

Other processes for production of hydrogen cyanide include the Fluohmic Process; by reacting ammonia and propane in an electrically heated fluidized carbon bed; ammoxidation of methanol or other hydrocarbons; and a decomposition of formamide, which is produced from ammonia and carbon monoxide. Any process that produces hydrogen cyanide in sufficient quantities can be used with this invention.

Under the process of this invention, an unpurified hydrogen cyanide-containing gas stream, or such a stream which has been treated to remove unreacted ammonia, is scrubbed with un aqueous solution of an inorganic acid suitable for use in nitrilotriacetonitrile synthesis. Preferred is an aqueous solution containing sulfuric acid, and preferably about 10% to about 25% apparent sulfuric acid concentration. Apparent sulfuric acid concentration is determined by titration against a standard sodium hydroxide solution. The unpurified gas stream may be cooled prior to scrubbing, preferably to about 80°C to about 100°C, or may be both cooled and treated to remove the unreacted ammonia. The resulting solution preferably contains about 10% to about 60%, more preferably about 20% to about 40%, hydrogen cyanide. This hydrogen cyanide-containing solution can be used without further purification to produce nitrilotriacetonitrile, using any suitable process for synthesizing nitrilotriacetonitrile.

Example 1

The scrubbing efficiency of hydrogen cyanide by a dilute sulfuric acid solution was determined at about 44°C and at about 32°C. Five percent hydrogen cyanide in an inert gas was bubbled at a rate of about 10 milliliters per minute into 100 milliliters of solution, through 5.5 centimeters of the solution, in a constant temperature water bath, until the scrubbed gas contained about 1% hydrogen cyanide. At about 44°C, the hydrogen cyanide concentration of the scrubbing solution reached about 25%, and at about 32°C, the hydrogen cyanide concentration of the solution reached about 57%.

Example 2

Nitrilotriacetonitrile was produced in a batch reaction in a sealed, rocking autoclave. The reaction mixture consisted of the following:

35 g $H_2O$
50 g HCN
13 g 03.5% $H_2SO_4$
53 g 51.3% $CH_2O$
62 g 33% HMTA

The water, hydrogen cyanide, and sulfuric acid represent a solution with 51% hydrogen cyanide, which is equivalent to a solution produced by scrubbing a hydrogen cyanide-containing gas stream at about 33°C with the water-sulfuric acid solution.

The entire reaction mixture was placed in an autoclave that had been cooled in a methanol ice bath and sealed. The sealed autoclave was placed on a heater/rocker, was rocked to provide agitation, and was heated to about 95°C. This temperature was maintained for about 40 minutes. The autoclave was cooled in an air stream to about 50—55°C, and was cooled in an ice bath to below 30°C. Nitrilotriacetonitrile was produced with a yield of 93.4% based upon the hexamethylene-tetramine, which was the limiting reactant.

Example 3

This example describes the process of this invention with the Degussa Process for production of hydrogen cyanide. Ammonia and methane are fed into an indirectly heated tubular reactor. The product gas stream is then cooled to 80°C to 100°C. This cooled gas stream contains about 71% hydrogen cyanide, with the remainder being methane, ammonia, hydrogen, and nitrogen. This cooled gas stream is then scrubbed with a solution of sulfuric acid with an apparent sulfuric acid concentration of about 18.5%. The hydrogen cyanide-free gas stream is removed, and the hydrogen cyanide-containing solution, with about 33% hydrogen cyanide is then used in the reaction with hexamethylenetetramine and formaldehyde or with ammonia and formaldehyde to produce nitrilotriacetonitrile.

Example 4

This example describes the process of this invention with the Andrussow Process, with ammonia scrubbing prior to the hydrogen cyanide scrubbing. Methane, ammonia, and air are fed into a reactor containing platinum-rhodium catalyst. The product gas stream is then cooled to 80°C to 100°C. This cooled gas stream is then scrubbed with a solution containing about 30% monoammonium phosphate. The

ammonium-containing solution could be treated to recover the ammonia, or could be used directly as diammonium phosphate. The ammonia-free gas stream is then scrubbed with a solution of sulfuric acid with an apparent sulfuric acid concentration of about 18.5%. The hydrogen cyanide-containing gas stream is removed, and the hydrogen cyanide-containing solution, with about 33% hydrogen cyanide is then used in the reaction with hexamethylenetetramine and formaldehyde or with ammonia and formaldehyde to produce nitrilotriacetonitrile.

**Claims**

1. A process for producing nitrilotriacetonitrile consisting essentially of:

a. producing a gas stream containing hydrogen cyanide;

b. cooling said gas to a temperature suitable for scrubbing;

c. scrubbing said cooled gas stream with a dilute aqueous solution of an inorganic acid to produce a hydrogen cyanide-containing solution;

d. adding formaldehyde and an ammonia derivative selected from the group consisting of ammonia, an ammonium derivative, and hexamethylenetetramine to said hydrogen cyanide-containing solution, to produce a reaction mixture; and

e. reacting said reaction mixture to produce nitrilotriacetonitrile.

2. The process of Claim 1 wherein the ammonia derivative is hexamethylenetetramine.

3. The process of Claim 1 wherein the ammonia derivative is ammonia or an ammonium salt.

4. The process of Claim 1 wherein the gas stream containing hydrogen cyanide is produced by reacting methane, ammonia, and oxygen in the presence of a metal catalyst from Group VIII of the Periodic Table.

5. The process of Claim 1 wherein the gas stream containing hydrogen cyanide is produced by reacting methane and ammonia in an indirectly heated reactor.

6. The process of Claim 1 wherein the gas stream containing hydrogen cyanide is produced by reacting ammonia and propane in an electrically heated fluidized carbon bed.

7. The process of Claim 1 wherein the aqueous scrubbing solution contains sulfuric acid.

8. The process of Claim 7 wherein the apparent concentration of sulfuric acid is about 10% to about 25%.

9. The process of Claim 1 wherein the hydrogen cyanide-containing solution contains about 10% to about 60% hydrogen cyanide.

10. The process of Claim 1 wherein the hydrogen cyanide-containing solution contains about 20% to about 40% hydrogen cyanide.

11. The process of Claim 1, further consisting of treating the gas stream containing hydrogen cyanide to remove ammonia, prior to scrubbing of (c).

**Patentansprüche**

1. verfahren zur Herstellung von Nitrilotriacetonitril, dadurch gekennzeichnet, daß man im wesentlichen

(a) einen Cyanwasserstoff enthaltenden Gasstrom erzeugt;

(b) das Gas auf eine für das Auswaschen geeignete Temperatur abkühlt;

(c) den abgekühlten Gasstrom mit einer verdünnten wäßrigen Lösung einer anorganischen Säure auswäscht unter Bildung einer Cyanwasserstoff enthaltenden Lösung;

(d) Formaldehyd und ein Ammoniakderivat ausgewählt aus der Ammoniak, Ammoniumderivate und Hexamethylentetramin umfassenden Gruppe zu der Cyanwasserstoff enthaltenden Lösung zusetzt unter Bildung einer Reaktionsmischung; and

(e) die Reaktionsmischung unter Bildung von Nitrilotriacetonitril umsetzt.

2. Verfahren nach Anspruch 1, dadurch gekennzeichnet, daß man als Ammoniakderivat Hexamethylentetramin einsetzt.

3. Verfahren nach Anspruch 1, dadurch gekennzeichnet, daß man als Ammoniakderivat Ammoniak oder ein Ammoniumsalz einsetzt.

4. Verfahren nach Anspruch 1, dadurch gekennzeichnet, daß der Cyanwasserstoff enthaltende Gasstrom durch Umsetzen von Methan, Ammoniak und Sauerstoff in Gegenwart eines Metallkatalysators aus der Gruppe VIII des Periodensystems gebildet wird.

5. Verfahren nach Anspruch 1, dadurch gekennzeichnet, daß der Cyanwasserstoff enthaltende Gasstrom durch Umsetzen von Methan und Ammoniak in einem indirekt geheizten Reaktor gebildet wird.

6. Verfahren nach Anspruch 1, dadurch gekennzeichnet, daß der Cyanwasserstoff enthaltende Gasstrom durch Umsetzen von Ammoniak und Propan in einer elektrisch geheizten Kohlenstoff-Wirbelschicht gebildet wird.

7. Verfahren nach Anspruch 1, dadurch gekennzeichnet, daß die wäßrige Waschlösung Schwefelsäure enthält.

8. Verfahren nach Anspruch 7, dadurch gekennzeichnet, daß die scheinbare Konzentration der Schwefelsäure etwa 10% bis etwa 25% beträgt.

9. Verfahren nach Anspruch 1, dadurch gekennzeichnet, daß die Cyanwasserstoff enthaltende Lösung etwa 10% bis etwa 60% Cyanwasserstoff enthält.

10. Verfahren nach Anspruch 1, dadurch gekennzeichnet, daß die Cyanwasserstoff enthaltende Lösung etwa 20% bis etwa 40% Cyanwasserstoff enthält.

11. Verfahren nach Anspruch 1, dadurch gekennzeichnet, daß man vor dem Auswaschen in der Stufe (c) den Cyanwasserstoff enthaltenden Gasstrom zur Entfernung von Ammoniak behandelt.

**Revendications**

1. Procédé de production de nitrilotriacétonitrile consistant essentiellement à:

a. produire un courant de gaz contenant de l'acide cyanhydrique;

b. refroidir ce gaz à une température convenable pour le lavage;

c. laver ce courant de gaz refroidi avec une solution aqueuse diluée d'un acide minéral pour produire une solution contenant de l'acide cyanhydrique;

d. ajouter du formaldéhyde et un dérivé de l'ammoniac choisis dans le groupe constitué de l'ammoniac, d'un dérivé de l'ammonium, et de l'hexaméthylénetétramine à cette solution contenant de l'acide cyanhydrique, pour produire un mélange réactionnel; et

e. faire réagir ce mélange réactionnel pour produire du nitrilotriacétonitrile.

2. Procédé selon la revendication 1, dans lequel le dérivé de l'ammoniac est l'hexaméthylènetétramine.

3. Procédé selon la revendication 1, dans lequel le dérivé de l'ammoniac est l'ammoniac ou un sel d'ammonium.

4. Procédé selon la revendication 1, dans lequel le courant gazeux contenant de l'acide cyanhydrique est produit en faisant réagir du méthane, de l'ammoniac et de l'oxygène en présence d'un catalyseur métallique du groupe VIII de la classification péiodique.

5. Procédé selon la revendication 1, dans lequel le courant gazeux contenant de l'acide cyanhydrique est produit en faisant réagir du méthane et de l'ammoniac dans un réacteur chauffé indirectement.

6. Procédé selon la revendication 1, dans lequel le courant gazeux contenant de l'acide cyanhydrique est produit en faisant réagir de l'ammoniac et du propane dans un lit de carbone fluidisé chauffé électriquement.

7. Procédé selon la revendication 1, dans lequel la solution de lavage aqueuse contient de l'acide sulfurique.

8. Procédé selon la revendication 7, dans lequel la concentration apparente de l'acide sulfurique est d'environ 10% à environ 25%.

9. Procédé selon la revendication 1, dans lequel la solution contenant de l'acide cyanhydrique contient environ 10% à environ 60% d'acide cyanhydrique.

10. Procédé selon la revendication 1, dans lequel la solution contenant de l'acide cyanhydrique contient environ 20% à environ 40% d'acide cyanhydrique.

11. Procédé selon la revendication 1, consistant en outre à traiter le courant gazeux contenant de l'acide cyanhydrique pour éliminer l'ammoniac avant le lavage de (c).